# EUROPÄISCHE PATENTSCHRIFT

(11) **EP 1 720 591 B1**
(45) Veröffentlichungstag und Bekanntmachung des Hinweises auf die Patenterteilung: **08.01.2020**
(21) Anmeldenummer: 05715512.9
(22) Anmeldetag: 24.02.2005
(51) Int. Cl.: A61M 15/00, B05B 12/08

(54) **ZERSTÄUBER**
ATOMISER
PULVERISATEUR

(30) Priorität: 24.02.2004 DE 102004009436
(43) Veröffentlichungstag der Anmeldung: 15.11.2006
(73) Patentinhaber: Boehringer Ingelheim International GmbH, 55216 Ingelheim (DE)
(72) Erfinder: WUTTKE, Gilbert, 44149 Dortmund (DE); KUNZE, Hubert, 44227 Dortmund (DE); THÖMMES, Ralf, 47877 Willich (DE)
(74) Vertreter: Von Rohr Patentanwälte Partnerschaft mbB
(86) Internationale Anmeldenummer: PCT/EP2005/001944
(87) Internationale Veröffentlichungsnummer: WO 2005/079895

(56) Entgegenhaltungen:
- US-A- 5 664 703
- US-A1- 2003 192 535
- US-A1- 2003 205 229
- US-B1- 6 234 366

## Beschreibung

Der vorliegende Erfindung betrifft einen Zerstäuber gemäß dem Oberbegriff des Anspruchs 1.

Ausgangspunkt der vorliegenden Erfindung ist ein unter dem Handelsnamen "Respimat" von der Boehringer Ingelheim KG angebotener Zerstäuber in Form eines Inhalators, wie im Grundprinzip in der WO 91/14468 A1 und in konkreter Ausgestaltung in der WO 97/12687 A1 (Fig. 6a, 6b) sowie in Fig. 1 und 2 der anliegenden Zeichnung dargestellt. Der Zerstäuber weist als Reservoir für ein zu zerstäubendes Fluid einen einsetzbaren Behälter mit dem Fluid und einen Druckerzeuger mit einer Antriebsfeder zur Förderung und Zerstäubung des Fluids auf.

Zur Vervollständigung der Offenbarung der vorliegenden Patentanmeldung wird vorsorglich auf den kompletten Offenbarungsgehalt sowohl der WO 91/14468 A1 als auch der WO 97/12687 A1 verwiesen. Generell bezieht sich die dortige Offenbarung bevorzugt auf einen Zerstäuber mit einem Federdruck von 5 bis 60 MPa, bevorzugt 10 bis 50 MPa auf das Fluid, mit Volumina pro Hub von 10 bis 50 µl, bevorzugt 10 bis 20 µl, ganz bevorzugt etwa 15 µl pro Hub, Teilchengrößen von bis zu 20 µm, bevorzugt 3 bis 10 µm. Ferner bezieht sich die dortige Offenbarung bevorzugt auf einen Zerstäuber mit zylinderähnlicher Form und einer Größe von etwa 9 cm bis etwa 15 cm in der Länge und etwa 2 cm bis etwa 5 cm in der Breite sowie von einer Düsen-Strahlfächerung von 20° bis 160°, bevorzugt von 80° bis 100°. Derartige Werte gelten auch für den Zerstäuber nach der Lehre der Erfindung als besonders bevorzugte Werte.

Durch Drehen eines Betätigungsteils in Form eines Gehäuseunterteils des Zerstäubers ist die Antriebsfeder spannbar und Fluid in eine Druckkammer des Druckerzeugers saugbar. Nach manueller Betätigung eines Sperrelements wird das Fluid in der Druckkammer von der Antriebsfeder unter Druck gesetzt und zerstäubt, also unter Bildung eines Aerosols ausgegeben. Beim Spannen einerseits und der dann folgenden Zerstäubung andererseits führt der Behälter jeweils eine Hubbewegung aus.

Der Zerstäuber weist eine mechanische Überwachungseinrichtung auf, die zur Zählung von Betätigungen des Zerstäubers das Drehen des Betätigungsteils erfaßt, Der bekannte Zerstäuber arbeitet ausschließlich mechanisch, d. h. ohne Treibgas und ohne Elektrik.

Die US 6,234,366 betrifft einen Dispenser mit einer Steuereinheit zur zeitabhängigen Sperrung und Freigabe der Betätigung. Eine von einem Steuersignal betätigte Schaltung kann ein akustisches oder vibratorisches Signal auslösen. Innerhalb der Steuereinheit kann eine Spiralfeder als Energiespeicher für einen Steuerantrieb vorgesehen sein.

Der vorliegenden Erfindung liegt die Aufgabe zugrunde, einen Zerstäuber mit verbesserter Benutzerführung anzugeben.

Die obige Aufgabe wird durch einen Zerstäuber gemäß Anspruch 1 gelöst. Vorteilhafte Weiterbildungen sind Gegenstand der Unteransprüche.

Eine grundlegende Idee der vorliegenden Erfindung liegt darin, eine Signaleinrichtung zur Erzeugung mindestens eines akustischen und/oder vibratorischen Signals zur Benutzerführung vorzusehen. Dies ermöglicht eine verbesserte Benutzerführung, insbesondere auch während des Inhalierens, so daß eine verbesserte Handhabung und Sicherheit bei der Benutzung ermöglicht werden.

Bei der vorliegenden Erfindung ist der Begriff "vibratorisches Signal" in einem weiteren Sinne vorzugsweise auch dahingehend zu verstehen, daß er ein sonstiges taktiles Signal, wie die Bewegung eines Teils des Zerstäubers, ein Bewegen oder Entsperren eines Betätigungselements oder dergleichen, umfaßt.

Insbesondere weist der Zerstäuber ein Mundstück auf, so daß der Benutzer zur Benutzung des Zerstäubers das Mundstück in den Mund nimmt. Während des Zerstäubungsvorgangs und Inhalationsvorgangs ist dann eine eventuell vorhandene optische Anzeige am Zerstäuber für den Benutzer nicht mehr ablesbar. Ein akustisches und/oder vibratorisches Signal kann vom Benutzer jedoch auch während des Inhalationsvorgangs registriert werden.

Ein besonderer Vorteil eines vibratorischen Signals liegt darin, daß bei Aufnahme eines Mundstücks von einem Benutzer ein vibratorisches Signal verhältnismäßig gering sein kann, also eine kleine Amplitude aufweisen kann, und trotzdem sehr gut vom Benutzer registrierbar ist.

Alternativ oder zusätzlich zur Erfassung des vibratorischen Signals über ein Mundstück kann der Zerstäuber auch derart ausgebildet sein, daß das vibratorische Signal mittels der den Zerstäuber haltenden Hand, insbesondere eines Fingers, vom Benutzter erfaßt bzw. registriert werden kann. Hierbei kann es dann genügen, daß beispielsweise nur ein Abschnitt oder Teil, wie eine Auslösetaste, des Zerstäubers vibriert oder ein sonstiges taktiles Signal, wie eine Bewegung, erzeugt.

Ein weiterer Vorteil liegt in der Diskretion, da der Benutzer ein vibratorisches Signal erfassen kann, ohne daß das Signal von Dritten wahrgenommen wird.

Gemäß einer Ausführungsvariante ist ein Signal während der Dauer des Zerstäubungsvorgangs und/oder am Ende des Zerstäubungsvorgangs erzeugbar, um einen Benutzer entsprechend zu informieren.

Alternativ oder zusätzlich ist beginnend mit einem Zerstäubungsvorgang während einer vorgebbaren Zeitdauer und/oder nach Ablauf dieser Zeitdauer ein Signal erzeugbar. Dieses Signal zeigt einem Benutzer die ideale oder erforderliche Inhalationsdauer oder die Zeitdauer, während der ein Benutzer unmittelbar nach dem Inhalieren die Luft anhalten soll, bzw. deren Ende bei entsprechender Wahl dieser Zeitdauer - beispielsweise 1 bis 15 Sekunden länger als der Zerstäubungsvorgang - an.

Zusätzlich oder alternativ kann während der Dauer eines tatsächlichen Inhalationsvorgangs und/oder am Ende eines tatsächlichen Inhalationsvorgangs ein Signal erzeugt werden, wobei das Signal auch von der Stärke der Inhalation abhängen und ggf. ein ausreichend starkes oder ein nicht ausreichend starkes Inhalieren anzeigen kann. Insbesondere weist hierbei der Zerstäuber einen Sensor im Bereich eines Mundstücks des Zerstäubers auf, um einen beim Inhalieren vom Benutzer angesaugten Zuluftstrom zu erfassen und dadurch das Inhalieren zu detektieren.

Vorzugsweise zeigt der Zerstäuber mittels des Signals oder unterschiedlicher Signale dem Benutzer den Beginn, das Andauern und/oder das Ende des Zerstäubungsvorgangs, einer sich anschließenden - idealen oder tatsächlichen - Inhalationsdauer und/oder einer gewünschten Zeitdauer, vorzugsweise von etwa 5 bis 15 Sekunden, während der ein Benutzer unmittelbar nach dem Inhalieren die Luft anhalten soll, an.

Erfindungsgemäß arbeitet die Signaleinrichtung ebenso wie der Druckerzeuger ausschließlich mechanisch, also ohne Elektrik, Treibgas oder dgl.

Weitere Vorteile, Merkmale, Eigenschaften und Aspekte der vorliegenden Erfindung ergeben sich aus der folgenden Beschreibung bevorzugter Ausführungsformen anhand der Zeichnung. Es zeigt:
- Fig. 1: einen schematischen Schnitt eines bekannten Zerstäubers im ungespannten Zustand;
- Fig. 2: einen schematischen, um 90° gegenüber Fig. 1 gedrehten Schnitt des bekannten Zerstäubers im gespannten Zustand;
- Fig. 3: eine ausschnittsweise, schematische Schnittdarstellung eines vorschlagsgemäßen Zerstäubers gemäß einer ersten Ausführungsform mit einer Signaleinrichtung im gespannten Zustand;
- Fig. 4: einen Ausschnitt von Fig. 3 in vergrößerter Darstellung bei entspannter Signaleinrichtung;
- Fig. 5: eine ausschnittsweise, schematische Schnittdarstellung eines Zerstäubers gemäß einer zweiten Ausführungsform, die keinen Teil der beanspruchten Erfindung bildet und lediglich das Verständnis der Erfindung erleichtern soll, mit einer Signaleinrichtung im gespannten Zustand; und
- Fig. 6: einen vergrößerten Ausschnitt von Fig. 5.

In den Figuren werden für gleiche oder ähnliche Teile dieselben Bezugszeichen verwendet, wobei entsprechende oder vergleichbare Eigenschaften und Vorteile erreicht werden, auch wenn eine wiederholte Beschreibung weggelassen ist.

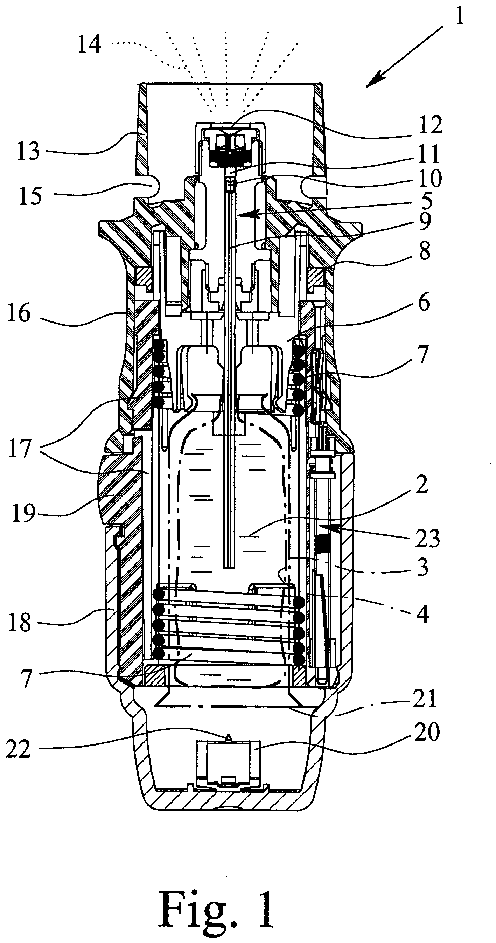

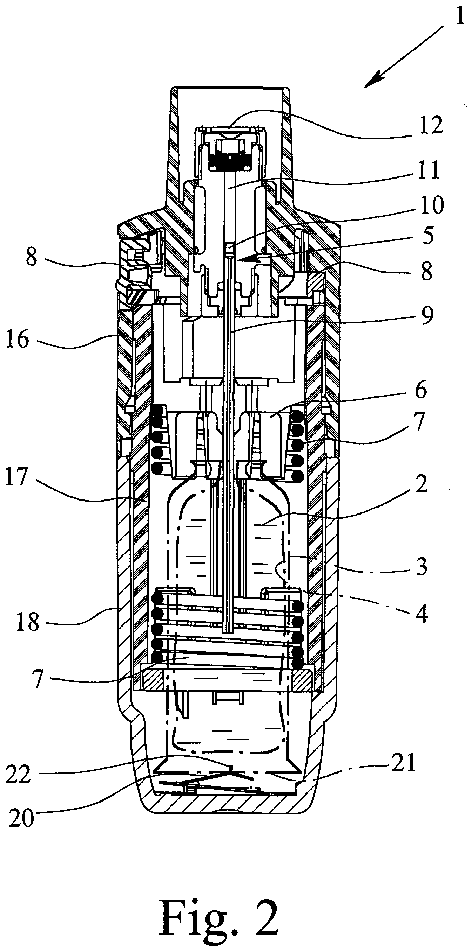

Fig. 1 und 2 zeigen einen bekannten Zerstäuber 1 zur Zerstäubung eines Fluids 2, insbesondere eines hochwirksamen Arzneimittels oder dgl., in einer schematischen Darstellung im ungespannten Zustand (Fig. 1) und gespannten Zustand (Fig. 2). Der Zerstäuber 1 ist insbesondere als tragbarer Inhalator ausgebildet und arbeitet vorzugsweise ohne Treibgas.

Bei Zerstäubung des Fluids 2, vorzugsweise einer Flüssigkeit, insbesondere eines Arzneimittels, wird ein Aerosol gebildet, das von einem nicht dargestellten Benutzer eingeatmet bzw. inhaliert werden kann. Üblicherweise erfolgt das Inhalieren wenigstens einmal täglich, insbesondere mehrmals täglich, vorzugsweise in vorbestimmten Zeitabständen.

Der Zerstäuber 1 weist einen einsetzbaren und vorzugsweise wechselbaren Behälter 3 mit dem Fluid 2 auf, der ein Reservoir für das zu zerstäubende Fluid 2 bildet. Vorzugsweise enthält der Behälter 3 eine ausreichende Menge an Fluid 2 für eine mehrfache Anwendung, insbesondere für eine vorbestimmte Applikationszeit, wie einen Monat, oder für mindestens 50, vorzugsweise mindestens 100, Dosierungen bzw. Zerstäubungen.

Der Behälter 3 ist im wesentlichen zylindrisch bzw. kartuschenartig ausgebildet und von unten, nach Öffnen des Zerstäubers 1, in diesen einsetzbar und ggf. wechselbar. Er ist vorzugsweise starr ausgebildet, insbesondere wobei das Fluid 2 in einem Beutel 4 im Behälter 3 aufgenommen ist.

Der Zerstäuber 1 weist einen Druckerzeuger 5 zur Förderung und Zerstäubung des Fluids 2, insbesondere jeweils in einer vorbestimmten, ggf. einstellbaren Dosiermenge, auf. Der Druckerzeuger 5 weist eine Halterung 6 für den Behälter 3, eine zugeordnete, nur teilweise dargestellte Antriebsfeder 7 mit einem zur Entsperrung manuell betätigbaren Sperrelement 8, ein Förderrohr 9 mit einem Rückschlagventil 10, eine Druckkammer 11 und eine Austragsdüse 12 im Bereich eines Mundstücks 13 auf.

Beim axialen Spannen der Antriebsfeder 7 wird die Halterung 6 mit dem Behälter 3 und dem Förderrohr 9 bei den Darstellungen nach unten bewegt und Fluid 2 aus dem Behälter 3 in die Druckkammer 11 des Druckerzeugers 5 über das Rückschlagventil 10 gesaugt. Da die Austragsdüse 12 einen sehr geringen Strömungsquerschnitt hat und insbesondere als Kapillare ausgebildet ist, ergibt sich eine so starke Drosselwirkung, daß auch ohne Rückschlagventil an dieser Stelle ein Einsaugen von Luft sicher ausgeschlossen ist.

Beim anschließenden Entspannen nach Betätigung des Sperrelements 8 wird das Fluid 2 in der Druckkammer 11 von der das Förderrohr 9 wieder nach oben bewegenden Antriebsfeder 7 - also durch Federkraft - unter Druck gesetzt und über die Austragsdüse 12 ausgegeben, wobei es zerstäubt wird, insbesondere in Partikel im µm- oder nm-Bereich, vorzugsweise lungengängige Partikel mit etwa 5 µm, die eine Wolke bzw. einen Strahl eines Aerosols 14 bilden, wie in Fig. 1 angedeutet. Die Förderung und Zerstäubung des Fluids 2 erfolgen vorzugsweise also rein mechanisch, insbesondere ohne Treibgas und ohne Elektrik.

Ein nicht dargestellter Benutzer kann das Aerosol 14 inhalieren, wobei Zuluft über mindestens eine Zuluftöffnung 15 in das Mundstück 13 saugbar ist.

Der Zerstäuber 1 weist ein Gehäuseoberteil 16 und ein demgegenüber drehbares Innenteil 17 auf, an dem ein insbesondere manuell betätigbares Gehäuseteil 18 vorzugsweise mittels eines Halteelementes 19 lösbar befestigt, insbesondere aufgesteckt, ist. Zum Einsetzen und/oder Auswechseln des Behälters 3 ist das Gehäuseteil 18 vom Zerstäuber 1 lösbar.

Durch manuelles Drehen des Gehäuseteils 18 ist das Innenteil 17 relativ zum Gehäuseoberteil 16 drehbar, wodurch die Antriebsfeder 7 über ein nicht dargestelltes, auf die Halterung 6 wirkendes Getriebe in axialer Richtung spannbar ist. Beim Spannen wird der Behälter 3 axial nach unten bewegt, bis der Behälter 3 eine in Fig. 2 angedeutete Endlage im gespannten Zustand annimmt. Während des Zerstäubungsvorgangs wird der Behälter 3 von der Antriebsfeder 7 wieder in seine Ausgangslage zurückbewegt. Der Behälter 3 führt also eine Hubbewegung während des Spannvorgangs und während des Zerstäubungsvorgangs aus.

Das Gehäuseteil 18 bildet vorzugsweise ein kappenartiges Gehäuseunterteil und um- bzw. übergreift einen unteren freien Endbereich des Behälters 3. Beim Spannen der Antriebsfeder 7 bewegt sich der Behälter 3 mit seinem Endbereich (weiter) in das Gehäuseteil 18 bzw. zu dessen stirnseitigem Ende hin, wobei eine axial wirkende, im Gehäuseteil 18 angeordnete Feder 20 am Behälterboden 21 zur Anlage kommt und mit einem Anstechelement 22 den Behälter 3 bzw. eine bodenseitige Versiegelung bei der erstmaligen Anlage zur Belüftung ansticht.

Der Zerstäuber 1 weist eine Überwachungseinrichtung 23 auf, die Betätigungen des Zerstäubers 1 zählt, vorzugsweise indem sie ein Drehen des Innenteils 17 zum Gehäuseoberteil 16 erfaßt. Die Überwachungseinrichtung 23 arbeitet beim Darstellungsbeispiel rein mechanisch.

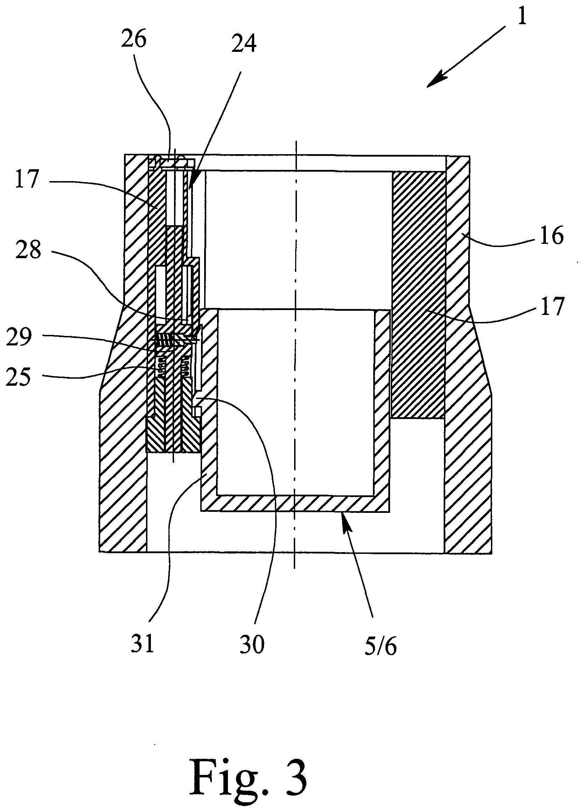

Nachfolgend werden der Aufbau und die Funktionsweise eines vorschlagsgemäßen Zerstäubers 1 näher erläutert, wobei auf die Fig. 3 bis 6 bezug genommen wird, jedoch nur wesentliche Unterschiede gegenüber dem Zerstäuber 1 gemäß Fig. 1 und 2 herausgestellt werden. Die Ausführungen zu Fig. 1 und 2 gelten also entsprechend.

Fig. 3 zeigt in einer sehr schematischen, nur ausschnittsweisen Schnittdarstellung einen vorschlagsgemäßen Zerstäuber 1 gemäß einer ersten Ausführungsform mit einer Signaleinrichtung 24 zur Erzeugung mindestens eines akustischen und/oder vibratorischen bzw. sonstigen taktilen Signals zur Benutzerführung bei Benutzung des Zerstäubers 1.

Die Signaleinrichtung 24 arbeitet vorzugsweise ausschließlich mechanisch. Gemäß anderer Varianten, die nicht Teil der beanspruchten Erfindung sind, kann die Signaleinrichtung 24 jedoch auch elektrisch oder beispielsweise pneumatisch arbeiten.

Die Signaleinrichtung 24 ist vom Druckerzeuger 5, insbesondere dessen Antriebsfeder 7, antreibbar. Die Signaleinrichtung 24 weist einen vom Druckerzeuger 5 unabhängigen Energiespeicher oder Antrieb, insbesondere einen Federspeicher 25, auf. Beim Darstellungsbeispiel ist der Federspeicher 25 durch eine Schraubenfeder gebildet.

Die Signaleinrichtung 24 bzw. deren Federspeicher 25 ist zusammen mit dem Druckerzeuger 5 von einem Teil, wie der Halterung 6 des Druckerzeugers 5, spannbar, insbesondere durch Drehen des Gehäuseteils 18 und damit des Innenteils 17.

Bei der ersten Ausführungsform ist die Signaleinrichtung 24 vorzugsweise in oder am insbesondere zum Spannen des Zerstäubers 1 drehbaren Innenteil 17 angeordnet. Jedoch kann die Signaleinrichtung 24 auch an oder in einem sonstigen geeigneten Teil des Zerstäubers 1 angeordnet sein.

Die Signaleinrichtung 24 weist ein vorzugsweise plattenförmiges Prallelement 26 und einen Stößel 27 oder dgl. zum Betätigen des Prallelements 26 auf. An Stelle des Prallelements 26 kann auch jedes sonstige geeignete Teil zur Erzeugung eines akustischen und/oder vibratorischen Signals bei Aufprall des Stößels 27 verwendet werden.

Der Federspeicher 25 spannt den Stößel 27 zum Prallelement 26 hin vor. Fig. 3 zeigt die Signaleinrichtung 24 im gespannten Zustand; der Stößel 27 ist also vom Prallelement 26 abgerückt.

Das Spannen des Stößels 27 gegen die Kraft des Federspeichers 25 erfolgt beim Darstellungsbeispiel zusammen mit dem Spannen des Druckerzeugers 5. Insbesondere greift eine Spannocke 28, die an einem axialbeweglichen Teil des Druckerzeugers 5, insbesondere der Halterung 6 oder einem damit verbundenen Teil, angeordnet ist, an dem Stößel 27 an, wie in Fig. 3 angedeutet. Der Druckerzeuger 5 und die Signaleinrichtung 24 werden also vorzugsweise in der gleichen Richtung gespannt.

Der Stößel 27 ist im gespannten Zustand mittels eines vorzugsweise stiftförmigen Sperrteils 29 sperrbar. Das Sperrteil 29 ist vorzugsweise federbelastet und nimmt bei Erreichen der gespannten Lage selbsttätig seine Sperrstellung ein, die in Fig. 3 gezeigt ist.

Die Signaleinrichtung 24 kann vom Druckerzeuger 5, insbesondere zu Beginn und/oder am Ende eines Zerstäubungsvorgangs, auslösbar oder einschaltbar sein.

Beim Darstellungsbeispiel ist die Signaleinrichtung 24 erst am Ende eines Zerstäubungsvorgangs auslösbar bzw. einschaltbar. Insbesondere erfolgt dies durch den Druckerzeuger 5 kurz vor oder bei Erreichen seines entspannten Zustands. Insbesondere entsperrt eine Auslösenocke 30 die Signaleinrichtung 24, indem die Auslösenocke 30 auf das Sperrteil 29 aufläuft und dieses in einen entsperrten Zustand drückt, so daß der Stößel 27 durch die Kraft des Federspeichers 25 zum Prallelement 26 bewegt, insbesondere beschleunigt, wird und auf das Prallelement 26 unter Erzeugung des gewünschten Signals prallt.

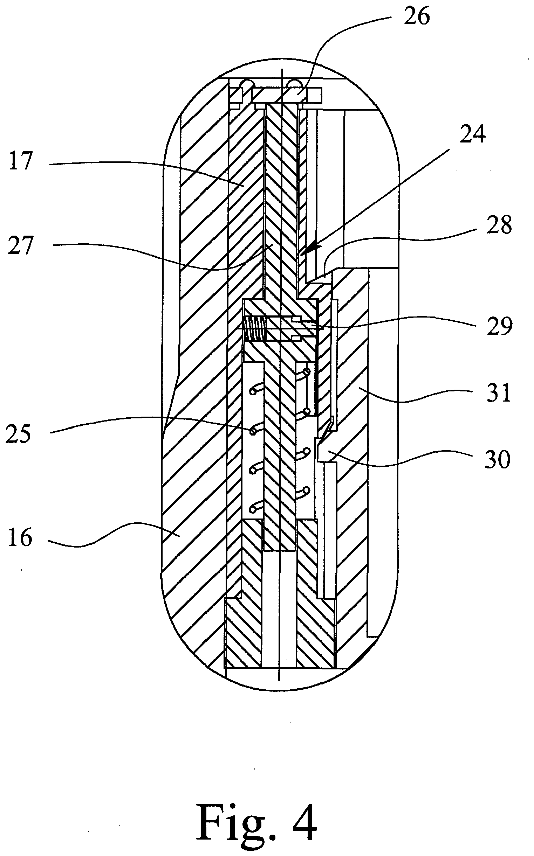

Die schematische, ausschnittsweise Schnittdarstellung gemäß Fig. 4 des Zerstäubers 1 zeigt die Signaleinrichtung 24 im entspannten Zustand, also bei oder nach Erzeugung des Signals.

Vorzugsweise sind die Spannocke 28 und die Auslösenocke 30 an einem gemeinsamen Nockenschieber 31 angeordnet bzw. ausgebildet, wie in Fig. 3 gezeigt.

Vorzugsweise ist der Nockenschieber 31 zum Spannen des Druckerzeugers 5 und während des Zerstäubungsvorgangs bewegbar.

Bei der ersten Ausführungsform erzeugt die Signaleinrichtung 24 am Ende eines Zerstäubungsvorgangs ein Signal, um einem nicht dargestellten Benutzer das Ende des Zerstäubungsvorgangs anzuzeigen. Insbesondere wird die Signaleinrichtung 24 vom Druckerzeuger 5 am Ende eines Zerstäubungsvorgangs ausgelöst bzw. eingeschaltet.

Alternativ oder zusätzlich kann die Signaleinrichtung 24 vom Druckerzeuger 5 zu Beginn eines Zerstäubungsvorgangs ausgelöst bzw. eingeschaltet werden. Dies ist insbesondere dann vorteilhaft, wenn während des gesamten Zerstäubungsvorgangs ein Signal erzeugt wird, das dem Benutzer den Zerstäubungsvorgang anzeigt. Beispielsweise kann dies dadurch erfolgen, daß die Signaleinrichtung 24, beispielsweise mittels des Stößels 27 oder einem nicht dargestellten Kolben, einen Luftstrom erzeugt, der seinerseits das gewünschte Signal - beispielsweise mittels einer nicht dargestellten Pfeife oder dgl. - erzeugt.

Die Signaleinrichtung 24 kann also zusätzlich oder alternativ auch während der Dauer eines Zerstäubungsvorgangs ein Signal erzeugen.

Gemäß einer nicht dargestellten Ausführungsvariante sind vorzugsweise unterschiedliche Signale, beispielsweise zur Anzeige des (noch andauernden) Zerstäubungsvorgangs und zur Anzeige eines Endes des Zerstäubungsvorgangs erzeugbar.

Wenn von der Signaleinrichtung 24 unterschiedliche Signale erzeugbar sind, unterscheiden sich diese vorzugsweise hinsichtlich ihres Klangs, ihrer Dauer, ihrer Lautstärke, ihrer Tonhöhe oder dgl.

Gemäß einer nicht dargestellten Ausführungsvariante ist die Signaleinrichtung 24 derart ausgebildet, daß wahlweise akustische oder vibratorische Signale erzeugbar sind. Dies ist einer universellen Einsetzbarkeit des Zerstäubers 1 zuträglich. Wenn mittels der Signaleinrichtung 24 vibratorische Signale erzeugbar sind, ist der Zerstäuber 1 insbesondere auch für Gehörlose einsetzbar bzw. leichter benutzbar. Vorzugsweise ist die Signalerzeugung bedarfsweise ausschaltbar und/oder vom Benutzer vorgebbar, ob ein akustisches Signal, ein vibratorisches Signal oder beide Signale von der Signaleinrichtung 24 ausgebbar sind.

Der Zerstäubungsvorgang dauert üblicherweise etwa 1 bis 2 Sekunden. Der Inhalationsvorgang sollte jedoch noch etwa 1 bis 2 Sekunden länger fortgesetzt werden, um das zerstäubte Fluid 2 bzw. die erzeugte Wolke an Aerosol 14 möglichst vollständig zu inhalieren. Weiter sollte der Benutzer nach dem Einatmen bzw. Inhalieren die Luft für etwa 5 bis 15 Sekunden anhalten. Aus diesem Grund ist gemäß einer besonders bevorzugten, nicht dargestellten Ausführungsvariante vorgesehen, daß das Signal erst mit einer bestimmten Zeitverzögerung nach dem Ende des Zerstäubungsvorgangs und/oder noch eine gewisse Zeit lang nach Beendigung der Zerstäubung ausgegeben wird. Vorzugsweise ist also das Signal - insbesondere beginnend mit einem Zerstäubungsvorgang - während einer vorgebbaren Zeitdauer und/oder nach der vorgegebenen Zeitdauer ausgebbar, wobei die Zeitdauer vorzugsweise so bemessen ist, daß einem Benutzer die empfohlene bzw. erforderliche Inhalationszeit von beispielsweise etwa 3 bis 4 Sekunden oder das empfohlene Luftanhalten von etwas 5 bis 15 Sekunden anzeigbar ist.

Die vorgenannte Signalerzeugung kann beispielsweise dadurch erfolgen, daß die Signaleinrichtung 24 erst am Ende eines Zerstäubungsvorgangs - beispielsweise vom Druckerzeuger 5 - ausgelöst wird und dann ein Signal für eine Dauer von beispielsweise 1 bis 15 Sekunden oder erst nach 1 bis 15 Sekunden erzeugt. Dies ist beispielsweise durch die Erzeugung eines Luftstroms mittels des Stößels 27 zur unmittelbaren Erzeugung eines Signals oder zur Verlangsamung bzw. Dämpfung der Stößelbewegung zum Prallelement 26 hin realisierbar.

Gemäß einer Variante, die nicht zur beanspruchten Erfindung gehört, kann an Stelle einer mechanischen Verzögerung oder eines mechanischen Zeitglieds bedarfsweise auch ein elektronisches Zeitglied die genannte Verzögerung bzw. Zeitdauer zur Anzeige des empfohlenen bzw. erforderlichen Inhalationsvorgangs bzw. dessen Ende eingesetzt werden. In diesem Fall arbeitet die Signaleinrichtung vorzugsweise elektrisch bzw. elektronisch.

Gemäß einer weiteren Ausführungsvariante, die nicht Teil der beanspruchten Erfindung ist, kann die Signaleinrichtung 24 auch unabhängig vom Druckerzeuger 5 oder beispielsweise gleichzeitig durch Betätigung des Sperrelements 8 auslösbar sein.

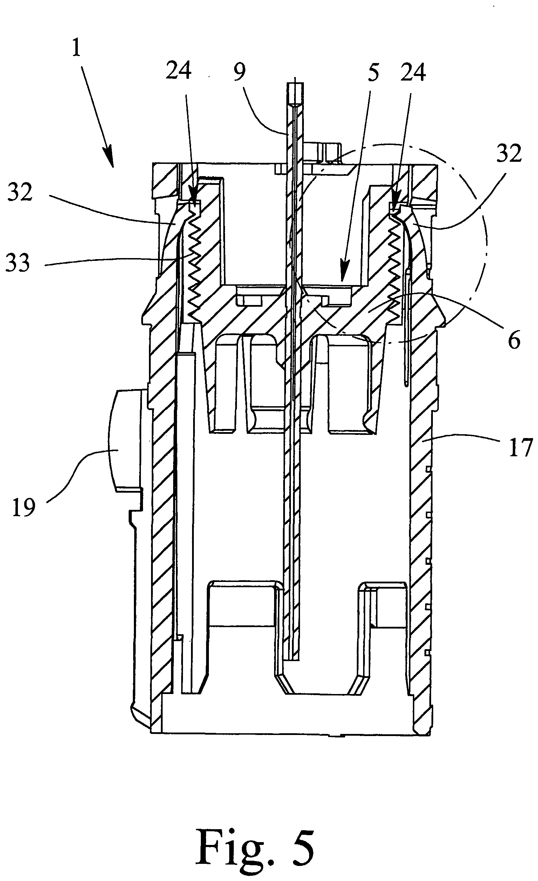

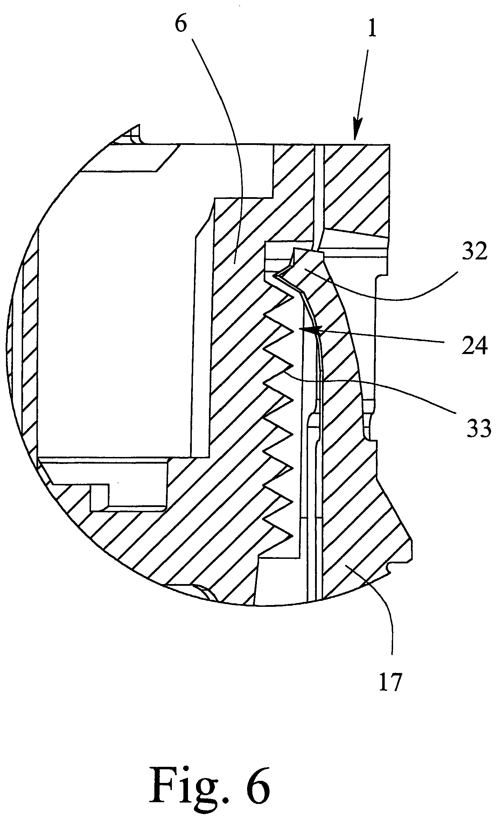

Fig. 5 zeigt in einer schematischen, ausschnittsweisen Schnittansicht eine nicht zur beanspruchten Erfindung gehörende, zweite Ausführungsform des Zerstäubers 1 mit einer anderen Signaleinrichtung 24. Fig. 6 zeigt eine ausschnittsweise Vergrößerung von Fig. 5.

Die Signaleinrichtung 24 weist mindestens ein Ratschenelement 32, beim Darstellungsbeispiel zwei Ratschenelemente 32, auf, das bzw. die während eines Zerstäubungsvorgangs über eine Rastung 33 unter Erzeugung eines akustischen Signals bewegbar ist bzw. sind. Insbesondere erfolgt die Relativbewegung der Rastung 33 zu den Ratschenelementen 32 durch den Druckerzeuger 5 bzw. dessen Antriebsfeder 7 während des Zerstäubungsvorgangs.

Vorzugsweise ist die Rastung 33 an der Halterung 6 oder einem damit verbundenen Teil ausgebildet.

Die Ratschenelemente 32 sind vorzugsweise fingerartig ausgebildet und insbesondere am Innenteil 17 des Zerstäubers 1 angeordnet bzw. ausgebildet, vorzugsweise einstückig angeformt.

Die Ratschenelemente 32 sind vorzugsweise elastisch, insbesondere eigenelastisch, gegen die Rastung 33 vorgespannt.

Bei der zweiten Ausführungsform ist während eines Zerstäubungsvorgangs von der Signaleinrichtung 24 ein akustisches und/oder vibratorisches Signal aufgrund der sich über die Rastung 33 bewegenden Ratschenelemente 32 ausgebbar.

Die Rastung 33 kann durch ein Gewinde gebildet sein, so daß bei Verdrehen der Rastung 33 relativ zu den Ratschenelementen 32 während des Spannvorgangs die Erzeugung eines Signals vermieden werden kann. Alternativ kann die Vermeidung eines Signals beim Spannvorgang auch durch einen sonstigen Effekt oder eine sonstige konstruktive Lösung erreicht werden.

Die voranstehend beschriebenen Ausführungsformen - insbesondere einzelne Elemente und Aspekte der Ausführungsformen - können je nach Bedarf miteinander kombiniert und/oder kinematisch umgekehrt werden.

### Bezugszeichenliste

- 1: Zerstäuber
- 2: Fluid
- 3: Behälter
- 4: Beutel
- 5: Druckerzeuger
- 6: Halterung
- 7: Antriebsfeder
- 8: Sperrelement
- 9: Förderrohr
- 10: Rückschlagventil
- 11: Druckkammer
- 12: Austragsdüse
- 13: Mundstück
- 14: Aerosol
- 15: Zuluftöffnung
- 16: Gehäuseoberteil
- 17: Innenteil
- 18: Gehäuseteil (Unterteil)
- 19: Halteelement
- 20: Feder (im Gehäuseunterteil)
- 21: Behälterboden
- 22: Anstechelement
- 23: Überwachungseinrichtung
- 24: Signaleinrichtung
- 25: Federspeicher
- 26: Prallelement
- 27: Stößel
- 28: Spannocke
- 29: Sperrteil
- 30: Auslösenocke
- 31: Nockenschieber
- 32: Ratschenelement
- 33: Rastung

## Patentansprüche

1. Zerstäuber (1) für ein Fluid (2), mit einem Druckerzeuger (5) zur Förderung und/oder Zerstäubung des Fluids (2), wobei der Zerstäuber (1) eine Signaleinrichtung (24) zur Erzeugung mindestens eines akustischen und/oder vibratorischen Signals zur Benutzerführung aufweist, wobei die Signaleinrichtung (24) ausschließlich mechanisch arbeitet, und wobei die Signaleinrichtung (24) einen vom Druckerzeuger (5) unabhängigen Federspeicher (25) aufweist,
**dadurch gekennzeichnet,**
**dass** der Federspeicher (25) zusammen mit dem Druckerzeuger (5) spannbar und als Antrieb der Signaleinrichtung (24) ausgebildet ist.

2. Zerstäuber nach Anspruch 1, **dadurch gekennzeichnet, dass** der Federspeicher (25) von einem Teil, wie einer Halterung (6) des Druckerzeugers (5) für einen Behälter (3) mit dem Fluid (2), vorzugsweise manuell spannbar ist.

3. Zerstäuber nach einem der voranstehenden Ansprüche, **dadurch gekennzeichnet, daß** die Signaleinrichtung (24) derart ausgebildet ist, daß mehrere unterschiedliche Signale erzeugbar sind, und/oder daß die Signaleinrichtung (24) derart ausgebildet ist, daß wahlweise akustische und/oder vibratorische Signale erzeugbar sind und/oder daß die Signalerzeugung unterdrückbar oder abschaltbar ist, und/oder daß das Signal bzw. die Signale - insbesondere beginnend mit einem Zerstäubungsvorgang - während der Dauer des Zerstäubungsvorgangs, am Ende des Zerstäubungsvorgangs, während einer vorzugsweise vorgebbaren Zeitdauer und/oder nach der Zeitdauer erzeugbar ist bzw. sind.

4. Zerstäuber nach einem der voranstehenden Ansprüche, **dadurch gekennzeichnet, daß** die Signaleinrichtung (24) vom Druckerzeuger (5), insbesondere zu Beginn und/oder am Ende eines Zerstäubungsvorgangs, auslösbar oder einschaltbar ist, und/oder daß das Signal bzw. die Signale während der Dauer eines tatsächlichen Inhalationsvorgangs und/oder am Ende eines tatsächlichen Inhalationsvorgangs erzeugbar ist bzw, sind.

5. Zerstäuber nach einem der voranstehenden Ansprüche, **dadurch gekennzeichnet, daß** die Signaleinrichtung (24) vom Druckerzeuger (5), insbesondere dessen Antriebsfeder (7), antreibbar ist.

6. Zerstäuber nach einem der voranstehenden Ansprüche, **dadurch gekennzeichnet, daß** die Signaleinrichtung (24) ein vorzugsweise plattenförmiges Prallelement (26) aufweist, das zur Erzeugung des Signals, insbesondere mittels eines Stößels (27), betätigbar, vorzugsweise anschlagbar, ist.

7. Zerstäuber nach den Ansprüchen 5 und 6, **dadurch gekennzeichnet, daß** der Stößel (27) vom Federspeicher (25) gegen das Prallelement (26) zur Erzeugung des Signals bewegbar ist, insbesondere wobei der Stößel (27) gegen die Kraft des Federspeichers (25) zusammen mit dem Druckerzeuger (5) spannbar ist, insbesondere wobei während des Spannvorgangs eine Spannocke (28) am Stößel (27) angreift und den Federspeicher (25) spannt.

8. Zerstäuber nach Anspruch 6 oder 7, **dadurch gekennzeichnet, daß** der Stößel (27) im gespannten Zustand sperrbar ist, insbesondere wobei der Stößel (27) vom Druckerzeuger (5), insbesondere am Ende eines Zerstäubungsvorgangs, vorzugsweise mittels einer Auslösenocke (30), entsperrbar ist.

9. Zerstäuber nach den Ansprüchen 7 und 8, **dadurch gekennzeichnet, daß** die Spannocke (28) und die Auslösenocke (30) an einem gemeinsamen Nockenschieber (31) ausgebildet sind, insbesondere wobei der Nockenschieber (31) zum Spannen des Druckerzeugers (5) und während des Zerstäubungsvorgangs bewegbar ist.

10. Zerstäuber nach einem der voranstehenden Ansprüche, **dadurch gekennzeichnet, daß** die Signaleinrichtung (24) ein Ratschenelement (32) aufweist, das während eines Zerstäubungsvorgangs über eine Rastung (33) unter Erzeugung des Signals, insbesondere vom Druckerzeuger (5), bewegbar ist, insbesondere wobei die Rastung (33) an einer Halterung (6) des Druckerzeugers (5) für einen Behälter (3) mit dem Fluid (2) ausgebildet ist.

11. Zerstäuber nach Anspruch 10, **dadurch gekennzeichnet, daß** das Ratschenelement (32) fingerartig ausgebildet ist, und/oder daß das Ratschenelement (32) an einem Innenteil (17) des Zerstäubers (1) ausgebildet, insbesondere einstückig angeformt ist, und/oder daß das Ratschenelement (32) elastisch, insbesondere eigenelastisch, gegen die Rastung (33) vorgespannt ist.

12. Zerstäuber nach einem der voranstehenden Ansprüche, **dadurch gekennzeichnet, daß** der Druckerzeuger (5) nur mechanisch arbeitet und manuell betätigbar, insbesondere gegen Federkraft spannbar, ist, und/oder daß die Druckerzeugung bzw. Zerstäubung rein mechanisch, insbesondere treibgasfrei, vorzugsweise durch Federkraft, erfolgt.

13. Zerstäuber nach einem der voranstehenden Ansprüche, **dadurch gekennzeichnet, daß** der Zerstäuber (1) einen Behälter (3) aufweist, der das Fluid (2) enthält und während der Druckerzeugung bzw. Zerstäubung hubartig bewegbar ist, und/oder daß der Zerstäuber (1) als Inhalator, insbesondere zur medizinischen Aerosol-Therapie, ausgebildet ist.

## Claims

1. Nebulizer (1) for a fluid (2), having a pressure generator (5) for conveying and/or nebulizing the fluid (2), wherein the nebulizer (1) has a signal device (24) for generating at least one acoustic and/or vibratory signal for user guidance, wherein the signal device (24) operates exclusively mechanically, and wherein the signal device (24) has a spring store (25) which is independent of the pressure generator (5),
**characterised in that**
the spring store (25) can be (manually) tensioned together with the pressure generator (5) and is designed as a drive for the signal device (24).

2. Nebulizer according to claim 1, **characterized in that** the spring store (25) can preferably be clamped manually by a part, such as a holder (6) of the pressure generator (5) for a container (3) with the fluid (2).

3. Nebulizer according to one of the preceding claims, **characterized in that** the signal device (24) is designed in such a way that several different signals can be generated, and/or **in that** the signal device (24) is designed in such a way that acoustic and/or vibratory signals can be generated selectively and/or **in that** the signal generation can be suppressed or switched off, and/or **in that** the signal and/or the signals - in particular starting with a nebulizing process - can be generated during the duration of the nebulizing process, at the end of the nebulizing process, during a preferably predeterminable period of time and/or after the period of time.

4. Nebulizer according to one of the preceding claims, **characterized in that** the signal device (24) can be triggered or switched on by the pressure generator (5), in particular at the beginning and/or at the end of a nebulizing process, and/or **in that** the signal and/or signals can be generated during the duration of an actual inhalation process and/or at the end of an actual inhalation process.

5. Nebulizer according to one of the preceding claims, **characterized in that** the signal device (24) can be driven by the pressure generator (5), in particular its drive spring (7).

6. Nebulizer according to one of the preceding claims, **characterised in that** the signal device (24) has a preferably plate-shaped impact element (26) which can be actuated, preferably stopped, to generate the signal, in particular by means of a tappet (27).

7. Nebulizer according to claims 5 and 6, **characterized in that** the tappet (27) can be moved by the spring store (25) against the impact element (26) to generate the signal, in particular wherein the tappet (27) can be tensioned together with the pressure generator (5) against the force of the spring store (25), in particular wherein during the tensioning operation a tensioning cam (28) engages the tappet (27) and clamps the spring store (25).

8. Nebulizer according to claim 6 or 7, **characterized in that** the tappet (27) can be locked in the tensioned state, in particular wherein the tappet (27) can be unlocked by the pressure generator (5), in particular at the end of an nebulization process, preferably by means of a releasing cam (30).

9. Nebulizer according to claims 7 and 8, **characterized in that** the tensioning cam (28) and the releasing cam (30) are formed on a common cam slide (31), in particular wherein the cam slide (31) is movable for clamping the pressure generator (5) and during the nebulization process.

10. Nebulizer according to one of the preceding claims, **characterized in that** the signal device (24) has a ratchet element (32) which can be moved during a nebulization process via a latching (33) generating of the signal, in particular from the pressure generator (5), in particular wherein the latching (33) is formed on a holder (6) of the pressure generator (5) for a container (3) with the fluid (2).

11. Nebulizer according to claim 10, **characterized in that** the ratchet element (32) is formed finger-like, and/or **in that** the ratchet element (32) is formed on an inner part (17) of the nebulizer (1), in particular is preformed in onepiece, and/or **in that** the ratchet element (32) is elastically, in particular inherently elastic, preloaded against the latching (33).

12. Nebulizer according to one of the preceding claims, **characterised in that** the pressure generator (5) operates only mechanically and can be manually actuated, in particular tensioned against spring force, and/or **in that** the pressure generation and/or nebulization takes place purely mechanically, in particular propellant-gas-free, preferably by spring force.

13. Nebulizer according to one of the preceding claims, **characterized in that** the nebulizer (1) has a container (3) which contains the fluid (2) and is movable in a stroke-like manner during pressure generation and/or nebulization, and/or **in that** the nebulizer (1) is designed as an inhaler, in particular for medical aerosol therapy.

## Revendications

1. Pulvérisateur (1) pour un fluide (2), comprenant un générateur de pression (5) pour transporter et/ou pulvériser le fluide (2), le pulvérisateur (1) comprenant un dispositif de signalisation (24) pour générer au moins un signal acoustique et/ou vibratoire pour guider l'utilisateur, le dispositif de signalisation (24) fonctionnant exclusivement par la mécanique, le dispositif de signalisation (24) comprenant un ressort accumulateur (25), qui est indépendant du générateur de pression (5),
**caractérisé en ce que**
**en ce que** le ressort accumulateur (25) peut être tendu avec le générateur de pression (5) et est conçu comme un entraînement pour le dispositif de signalisation (24).

2. Pulvérisateur selon la revendication 1, **caractérisé en ce que** le ressort accumulateur (25) d'une pièce, tels qu'un support (6) du générateur de pression (5) pour un réservoir (3) peuvent être serrés avec le fluide (2), de préférence manuellement.

3. Pulvérisateur selon l'une des revendications précédentes, **caractérisé en ce que** le dispositif de signalisation (24) est conçu de telle sorte qu'une pluralité de signaux différents peut être générée, et/ou **en ce que** le dispositif de signalisation (24) est conçu de telle sorte que des signaux éventuellement acoustiques et/ou vibratoires peuvent être générés, et/ou **en ce que** la génération du signal peut être supprimée ou arrêtée, et/ou **en ce que** le et/ou les signaux - en particulier à partir d'un processus de pulvérisation - peut (peuvent) être généré(s) générés pendant la durée du processus de pulvérisation, à la fin du processus de pulvérisation, pendant une période de temps de préférence prédéfinissable et/ou après cette période.

4. Pulvérisateur selon l'une des revendications précédentes, **caractérisé en ce que** le dispositif de signalisation (24) peut être déclenché ou activé par le générateur de pression (5), en particulier au début et/ou à la fin d'un processus de pulvérisation, et/ou **en ce que** le ou les signaux peuvent être générés pendant la durée d'un processus d'inhalation réel et/ou à la fin d'un processus d'inhalation réel.

5. Pulvérisateur selon l'une des revendications précédentes, **caractérisé en ce que** le dispositif de signalisation (24) peut être entraîné par le générateur de pression (5), en particulier son ressort d'entraînement (7).

6. Pulvérisateur selon l'une des revendications précédentes, **caractérisé en ce que** le dispositif de signalisation (24) présente un élément d'impact (26) de préférence en forme de plaque qui peut être actionné, de préférence arrêtée, pour générer le signal, notamment au moyen d'un pilon (27).

7. Pulvérisateur selon les revendications 5 et 6, **caractérisé en ce que** le pilon (27) peut être déplacé par le ressort accumulateur (25) contre l'élément d'impact (26) pour générer le signal, en particulier dans lequel le pilon (27) peut être serré contre la force du ressort accumulateur (25) avec le générateur de pression (5), en particulier pendant le serrage une came de serrage (28) engage le pilon (27) et pince le ressort accumulateur (25).

8. Pulvérisateur selon la revendication 6 ou 7, **caractérisé en ce que** le pilon (27) peut être verrouillé à l'état serré, en particulier dans lequel le pilon (27) peut être déverrouillé par le générateur de pression (5), en particulier à la fin d'un processus de pulvérisation, de préférence par une came de libération (30).

9. Pulvérisateur selon les revendications 7 et 8, **caractérisé en ce que** la came de serrage (28) et la came de libération (30) sont formées sur un chariot à came (31) commun , en particulier le chariot à came (31) étant mobile pour serrer le générateur de pression (5) et pendant le processus de pulvérisation.

10. Pulvérisateur selon l'une des revendications précédentes, **caractérisé en ce que** le dispositif de signalisation (24) présente un élément à cliquet (32) qui peut être déplacé pendant un processus de pulvérisation via un crantage (33) avec génération du signal, notamment du générateur de pression (5), en particulier le crantage (33) étant formé sur un support (6) du générateur de pression (5) pour un réservoir (3) avec le fluide (2).

11. Pulvérisateur selon la revendication 10, **caractérisé en ce que** l'élément à cliquet (32) est en forme de doigt et/ou **en ce que** l'élément à cliquet (32) est formé sur une partie intérieure (17) du pulvérisateur (1), en particulier en une seule pièce, et/ou **en ce que** l'élément à cliquet (32) est sollicité contre le crantage (33) par un élasthème, notamment par un élasthénage.

12. Pulvérisateur selon l'une des exigences précédentes, **caractérisé en ce que** le générateur de pression (5) ne fonctionne que mécaniquement et est actionnable manuellement, en particulier peut être serré contre la/une force du ressort, et/ou **en ce que** la génération de pression ou la pulvérisation a lieu purement mécaniquement, en particulier sans gaz propulseur, de préférence par force élastique.

13. Pulvérisateur selon l'une des revendications précédentes, **caractérisé en ce que** le pulvérisateur (1) comporte un réservoir (3) qui contient le fluide (2) et qui peut être déplacé en forme de course lors de la génération de pression et/ou de la pulvérisation, et/ou **en ce que** le pulvérisateur (1) est conçu en tant qu'inhalateur, en particulier pour la thérapie aérosol médicale.
